(19) Europäisches Patentamt
European Patent Office
Office européen des brevets

(11) Veröffentlichungsnummer : **0 398 097 B1**

(12) # EUROPÄISCHE PATENTSCHRIFT

(45) Veröffentlichungstag der Patentschrift : **24.02.93 Patentblatt 93/08**

(51) Int. Cl.$^5$ : **C07C 337/06,** C07C 281/16, C07D 249/14

(21) Anmeldenummer : **90108483.0**

(22) Anmeldetag : **05.05.90**

(54) **Verfahren und neue Zwischenprodukte zur Herstellung von Triazolon-Derivaten.**

(30) Priorität : **18.05.89 DE 3916207**

(43) Veröffentlichungstag der Anmeldung : **22.11.90 Patentblatt 90/47**

(45) Bekanntmachung des Hinweises auf die Patenterteilung : **24.02.93 Patentblatt 93/08**

(84) Benannte Vertragsstaaten : **BE CH DE FR GB IT LI NL**

(56) Entgegenhaltungen :
**EP-A- 0 305 844**
**DE-A- 3 316 095**
**DE-B- 1 222 068**

(73) Patentinhaber : **BAYER AG**
**W-5090 Leverkusen 1 Bayerwerk (DE)**

(72) Erfinder : **Findeisen, Kurt, Dr.**
**Dünfelder Strasse 28**
**W-5090 Leverkusen 1 (DE)**
Erfinder : **Lindig, Markus, Dr.**
**Hildegardstr. 9**
**W-4018 Langenfeld (DE)**

EP 0 398 097 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren und neue Zwischenprodukte zur Herstellung von weitgehend bekannten Triazolon-Derivaten.

Es ist bekannt, daß 4-Methyl-5-methylamino-2,4-dihydro-3H-1,2,4-triazol-3-thion als Nebenprodukt bei der Umsetzung von 1,2-(Bis-methylamino-thiocarbonyl)-hydrazin mit Natriumacetat in wäßrigem Ethanol erhalten wird (vgl. J. Heterocycl. Chem. 15(1978), 377-384).

Es wurde nun gefunden, daß man Triazolon-Derivate der allgemeinen Formel (I)

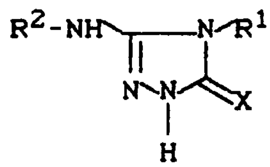

(I)

in welcher

$R^1$ für Alkyl, Alkenyl, Alkinyl, Halogenalkyl, Halogenalkenyl, Halogenalkinyl, Alkoxyalkyl, Cycloalkylalkyl, Cycloalkyl oder für jeweils gegebenenfalls substituiertes Aralkyl oder Aryl steht,

$R^2$ für Alkyl, Alkenyl, Alkinyl, Halogenalkyl, Halogenalkenyl, Halogenalkinyl, Alkoxyalkyl, Cycloalkyl, Cycloalkylalkyl oder für jeweils gegebenenfalls substituiertes Aryl, Aralkyl oder Heteroaryl steht und

X für Sauerstoff oder Schwefel steht,

in guten Ausbeuten und in hoher Reinheit erhält,

wenn man Iso(thio)cyanate der allgemeinen Formel (II)

$$X{=}C{=}N\text{-}R^1 \qquad \text{(II)}$$

in welcher

$R^1$ und X die oben angegebenen Bedeutungen haben,

mit Aminoguanidinen der allgemeinen Formel (III)

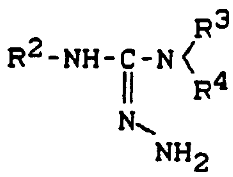

(III)

in welcher

$R^2$ die oben angegebene Bedeutung hat und

$R^3$ und $R^4$ unabhängig voneinander für Alkyl stehen,

oder mit Säureaddukten von Verbindungen der Formel (III)

gegebenenfalls in Gegenwart eines Verdünnungsmittels bei Temperaturen zwischen 0°C und 150°C umsetzt (Stufe 1) und die hierbei gebildeten (Thio)ureidoguanidine der allgemeinen Formel (IV)

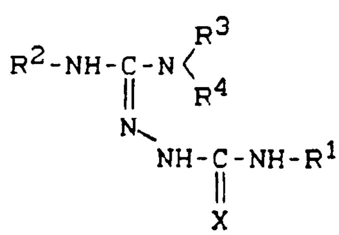

(IV)

in welcher

$R^1$, $R^2$, $R^3$, $R^4$ und X die oben angegebenen Bedeutungen haben,

oder gegebenenfalls Säureaddukte von Verbindungen der Formel (IV)

gegebenenfalls nach üblichen Methoden isoliert und gegebenenfalls auch ohne Zwischenisolierung ("in situ") weiter, gegebenenfalls in Gegenwart eines Säureakzeptors und gegebenenfalls in Gegenwart eines Verdünnungsmittels, unter Abspaltung eines Dialkylamins ($HNR^3R^4$) bei Temperaturen zwischen 0°C und 150°C zu den Verbindungen der Formel (I) kondensiert (Stufe 2).

Es ist als überraschend zu bezeichnen, daß nach dem erfindungsgemäßen Verfahren als Endprodukte praktisch nur die Triazolon-Derivate der Formel (I) unter Abspaltung von Aminen der Formel $HNR^3R^4$ erhalten werden, da auch alternativ ein Ringschluß der Zwischenprodukte der Formel (IV) unter Abspaltung von Aminen der Formel $R^2NH_2$ zu erwarten war.

Vorteile des erfindungsgemäßen Verfahrens liegen in der einfachen Zugänglichkeit der benötigten Ausgangsstoffe und in der umkomplizierten Verfahrensführung sowie in der guten Ausbeute und in der hohen Reinheit der Produkte.

Die nach dem erfindungsgemäßen Verfahren herzustellenden Triazolon-Derivate sind durch die Formel (I) allgemein definiert. Vorzugsweise werden nach dem erfindungsgemäßen Verfahren Verbindungen der Formel (I) hergestellt, in welcher

$R^1$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten infrage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und-/oder Schwefel steht, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, und

X für Sauerstoff oder Schwefel steht.

Besonders bevorzugt werden nach dem erfindungsgemäßen Verfahren Verbindungen der Formel (I) hergestellt, bei welchen

$R^1$ für Methyl, Ethyl, n- oder i-Propyl, n-, i-, s- oder t-Butyl, n- oder i-Pentyl, n- oder i-Hexyl, für Allyl, Propargyl, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio, und

X für Sauerstoff oder Schwefel steht.

Ganz besonders bevorzugt lassen sich die Verbindungen der Formel (I) herstellen, in welcher

$R^1$ für Methyl, Ethyl, n- oder iso-Propyl steht,

$R^2$ für Methyl, Ethyl, n- oder iso-Propyl, n-, iso-, sec.- oder tert.-Butyl oder Allyl steht und

X für Sauerstoff oder Schwefel steht.

Im einzelnen seien außer den bei den Herstellungsbeispielen genannten Verbindungen die folgenden Verbindungen der allgemeinen Formel (I) genannt:

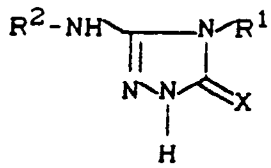

(I)

| $R^1$ | $R^2$ | X |
|---|---|---|
| $CH_3$ | $CH_3$ | O |
| $CH_3$ | $CH_3$ | S |
| $CH_3$ | $C_2H_5$ | O |
| $CH_3$ | $C_2H_5$ | S |
| $C_2H_5$ | $CH_3$ | O |
| $C_2H_5$ | $CH_3$ | S |
| $C_2H_5$ | $C_2H_5$ | O |
| $C_2H_5$ | $C_2H_5$ | S |
| $CH_2{=}CHCH_2$ | $CH_3$ | O |
| $CH_2{=}CHCH_2$ | $CH_3$ | S |

Verwendet man beispielsweise 1-Amino-2,2,3-trimethyl-guanidin-Hydrochlorid und Allylisocyanat als Ausgangsstoffe, so kann der Reaktionsablauf beim erfindungsgemäßen Verfahren durch das folgende Formelschema wiedergegeben werden:

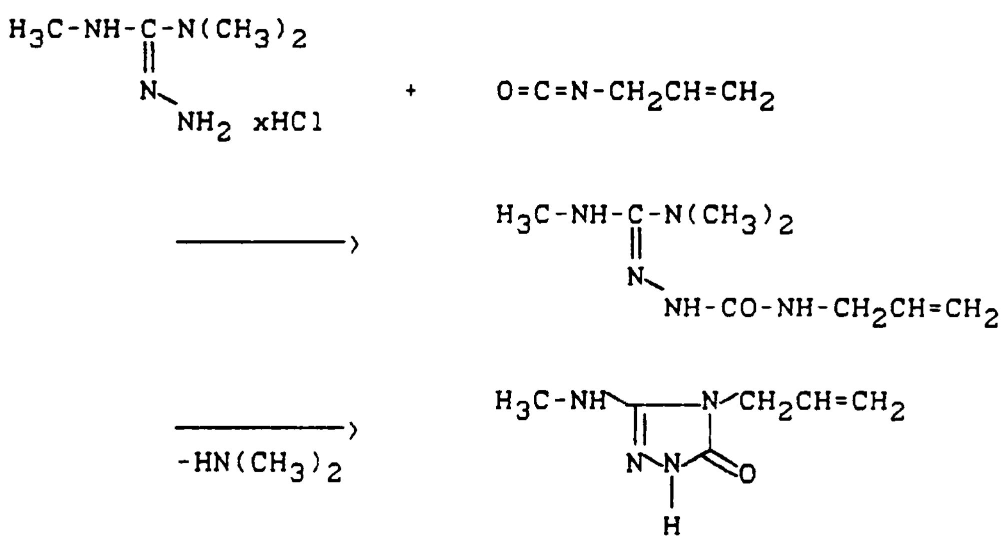


Die beim erfindungsgemäßen Verfahren zur Herstellung von Verbindungen der Formel (I) als Ausgangsstoffe zu verwendenden Iso(thio)cyanate sind durch die Formel (II) allgemein definiert.

In Formel (II) haben $R^1$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und X angegeben wurden.

Als Beispiele für die Ausgangsstoffe der Formel (II) seien genannt:

Methyl-, Ethyl-, Propyl-, Isopropyl-, Butyl-, Isobutyl-, sec-Butyl-, tert-Butyl-, Pentyl-, Isopentyl-, Hexyl-, Isohexyl-, Allyl-, Propargyl-, Methoxymethyl-, 2,2,2-Trifluorethyl-, Cyclopentyl-, Cyclohexyl-, Cyclopropyl-, Cyclopropylmethyl-, Cyclohexylmethyl- und Cyclohexylethyl-isocyanat und -isothiocyanat.

Die Ausgangsstoffe der Formel (II) sind bekannte organische Synthesechemikalien.

Die beim erfindungsgemäßen Verfahren zur Herstellung von Verbindungen der Formel (I) weiter als Ausgangsstoffe zu verwendenden Aminoguanidine sind durch die Formel (III) allgemein definiert.

In Formel (III) hat $R^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^2$ angegeben wurde und $R^3$ und $R^4$ stehen vorzugsweise für Methyl oder Ethyl, insbesondere für Methyl.

Als Beispiele für die Ausgangsstoffe der Formel (III) seien genannt:

1-Amino-2,2,3-trimethyl-, 1-Amino-2,2-dimethyl-3-ethyl-, 1-Amino-2,2-dimethyl-3-propyl-, 1-Amino-2,2-dimethyl-3-isopropyl-, 1-Amino-2,2-dimethyl-3-butyl-, 1-Amino-2,2-dimethyl-3-isobutyl-, 1-Amino-2,2-dimethyl-3-sec-butyl-, 1-Amino-2,2-dimethyl-3-tert-butyl-, 1-Amino-2,2-dimethyl-3-pentyl-, 1-Amino-2,2-dimethyl-3-isopentyl-, 1-Amino -2,2-dimethyl-3-allyl-, 1-Amino-2,2-dimethyl-3-propargyl-, 1-Amino-2,2-dimethyl-3-cyclopropyl-, 1-Amino-2,2-dimethyl-3-cyclopropylmethyl-, 1-Amino-2,2-dimethyl-3-cyclopentyl-, 1-Amino-2,2-dimethyl-3-cyclopentylmethyl-, 1-Amino-2,2-dimethyl-3-cyclohexyl-, 1-Amino-2,2-dimethyl-3-cyclohexylmethyl- und 1-Amino-2,2-dimethyl-3-cyclohexylethyl-guanidin, sowie die entsprechenden Hydrochloride.

Die Ausgangsstoffe der Formel (III) bzw. deren Säureaddukte sind bekannt und/oder können nach an sich bekannten Verfahren hergestellt werden (vgl. J.Org. Chem. 19 (1954), 1807; Bull. Soc. Chim. France 1975, 1649; EP-A 283876).

Die beim erfindungsgemäßen Verfahren als Zwischenprodukte isolierbaren (Thio)Ureidoguanidine der Formel (IV) sind noch nicht aus der Literatur bekannt und sind Gegenstand der vorliegenden Erfindung.

In Formel (IV) haben $R^1$, $R^2$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und X angegeben wurden und $R^3$ und $R^4$ stehen vorzugsweise für Methyl oder Ethyl, insbesondere für Methyl.

Im einzelnen seien außer den bei den Herstellungsbeispielen genannten Verbindungen die folgenden Verbindungen der allgemeinen Formel (IV) genannt:

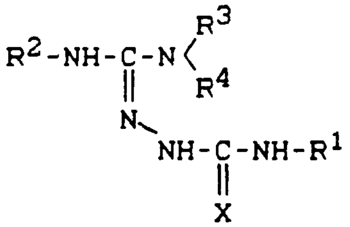

(IV)

| $R^1$ | $R^2$ | $R^3$ | $R^4$ | X |
|---|---|---|---|---|
| $CH_3$ | $CH_3$ | $CH_3$ | $CH_3$ | O |
| $CH_3$ | $CH_3$ | $CH_3$ | $CH_3$ | S |
| $CH_3$ | $C_2H_5$ | $CH_3$ | $CH_3$ | O |
| $CH_3$ | $C_2H_5$ | $CH_3$ | $CH_3$ | S |
| $C_2H_5$ | $CH_3$ | $CH_3$ | $CH_3$ | O |
| $C_2H_5$ | $CH_3$ | $CH_3$ | $CH_3$ | S |
| $C_2H_5$ | $C_2H_5$ | $CH_3$ | $CH_3$ | O |
| $C_2H_5$ | $C_2H_5$ | $CH_3$ | $CH_3$ | S |
| $CH_2=CHCH_2$ | $CH_3$ | $CH_3$ | $CH_3$ | O |
| $CH_2=CHCH_2$ | $CH_3$ | $CH_3$ | $CH_3$ | S |

Unter Säureaddukten sind im Zusammenhang mit der Definition der Verbindungen der Formeln (III) und (IV) Additionsprodukte dieser Verbindungen mit Säuren, insbesondere mit Mineralsäuren wie Hydrogenchlorid, Hydrogenbromid, Hydrogeniodid und Schwefelsäure, zu verstehen.

Das erfindungsgemäße Verfahren zur Herstellung der Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkali(hydrogen)carbonate und -alkoholate wie Natrium- und Kalium(hydrogen)carbonat, Natrium-und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Iso(thio)cyanat der Formel (II) im allgemeinen zwischen 0,8 und 1,2 Mol, vorzugsweise zwischen 0,9 und 1,1 Mol, Aminoguanidin der Formel (III) oder ein Säureaddukt hiervon ein.

Die Ausgangsstoffe der Formeln (II) und (III) werden im allgemeinen bei Raumtemperatur oder unter leichtem Kühlen zusammengegeben und dann, vorzugsweise bei erhöhter Temperatur, gerührt, bis die Reaktion der Ausgangsstoffe praktisch abgeschlossen ist. Die im allgemeinen kristallin anfallenden (Thio)Ureidoguanidine der Formel (IV) bzw. deren Säureaddukte können nach üblichen Methoden, z.B. durch Absaugen, isoliert werden. Sie können aber auch ohne Zwischenisolierung ("in situ") zu den Verbindungen der Formel (I) kondensiert werden. Hierfür wird vorzugsweise ein Säureakzeptor zum Reaktionsgemisch gegeben und die Mischung wird, vorzugsweise bei erhöhter Temperatur, bis zum Ende der Aminabspaltung gerührt.

Die Aufarbeitung kann nach üblichen Methoden durchgeführt werden. Im allgemeinen wird heiß filtriert und das im Filtrat beim Abkühlen kristallin anfallende Produkt der Formel (I) durch Absaugen isoliert. Alternativ wird nach der Heißfiltration das Filtrat eingeengt, der Rückstand mit Wasser und einem mit Wasser praktisch nicht mischbaren organischen Lösungsmittel, wie z.B. Methylenchlorid, geschüttelt, die organische Phase abgetrennt, mit einem Trockenmittel, wie z.B. Natriumsulfat getrocknet und filtriert. Vom Filtrat wird dann das Lösungsmittel im Vakuum sorgfältig abdestilliert. Der verbleibende Rückstand enthält im wesentlichen das Produkt der Formel (I).

Die Verbindungen der Formel (I) können beispielsweise als Additive bei der Elektroplattierung von Kupfer eingesetzt werden (vgl. JP 71-37646 = JP 46-37646, zitiert in Chem. Abstracts 77 (1972), 147067a).

Die Verbindungen der Formel (I) sind weiterhin wertvolle Zwischenprodukte zur Herstellung von Herbiziden der Formel (V)

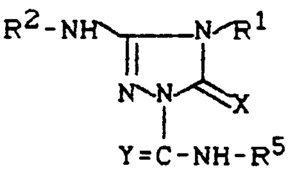

(V)

in welcher

$R^1$, $R^2$ und X die oben angegebene Bedeutung haben,

$R^5$ für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkylaminoalkyl, Alkoximinoalkyl, Alkoxycarbonylalkyl, Alkoxycarbonylalkenyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl oder Aryl, für Alkoxy, Alkenyloxy, Alkinyloxy, Aralkyloxy oder Aryloxy steht und

Y für Sauerstoff oder Schwefel steht.

Man erhält die herbizidwirksamen Verbindungen der Formel (V), wenn man Triazolon-Derivate der Formel (I)

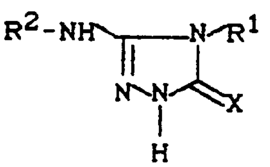

(I)

in welcher

$R^1$, $R^2$ und X die oben angegebene Bedeutung haben, mit Iso(thio)cyanaten der Formel (VI),

$$R^5\text{-N=C=Y} \qquad (VI)$$

in welcher

$R^5$ und Y die oben angegebenen Bedeutungen haben,

gegebenenfalls in Gegenwart eines Verdünnungsmittels wie beispielsweise Acetonitril und gegebenenfalls in Gegenwart eines Reaktionshilfsmittels wie beispielsweise Diazabicycloundecen (DBU) bei Temperaturen zwischen 0°C und +150°C umsetzt.

Herstellungsbeispiele:

Beispiel 1

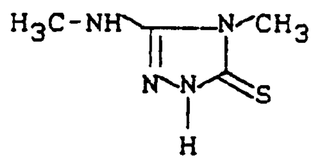

Stufe 1:

21,9 g (0,3 Mol) Methylisothiocyanat werden zu einer Mischung aus 45,75 g (0,3 Mol) 1-Amino-2,2,3-trimethyl-guanidin-Hydrochlorid und 300 ml Acetonitril gegeben und das Gemisch wird 2 Stunden bei Rückflußtemperatur gerührt. Nach Abkühlen wird das kristallin angefallene Produkt durch Absaugen isoliert. Man erhält 55,0 g (81% der Theorie) 1-(Methylamino-thiocarbonylamino)-2,2,3-trimethyl-guanidin-hydrochlorid nachstehender Formel.

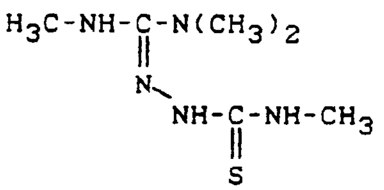

x HCl

$^1$H-NMR(DMSO-$D_6$, δ, ppm): 2,80-2,82; 2,85-2,87; 2,95.
MS: $M^+$ = 189.

Stufe 2:

55,0 g (0,245 Mol) 1-(Methylamino-thiocarbonylamino)-2,2,3-trimethyl-guanidin-Hydrochlorid werden in 300 ml Acetonitril mit 33,6 g (0,40 Mol) Natriumhydrogencarbonat 40 Minuten bei Rückflußtemperatur gerührt. Dann wird bei 70°C filtriert und das beim Erkalten des Filtrats und Einengen auf 1/4 des Volumens auskristallisierte Produkt wird durch Absaugen isoliert. Man erhält 32 g (74% der Theorie bezogen auf die Ausgangsstoffe der Stufe 1 bzw. 91% der Theorie bezogen auf das Ausgangsprodukt der Stufe 2) 4-Methyl-5-methylamino-2,4-dihydro-3H-1,2,4-triazol-3-thion vom Schmelzpunkt 213°C.
$^1$H-NMR (DMSO-$D_6$, δ, ppm): 2,68-2,73; 3,27.
MS: $M^+$ = 144.

Beispiel 2

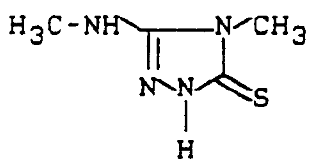

(Eintopfverfahren)

Eine Mischung aus 45,75 g (0,3 Mol) 1-Amino-2,2,3-trimethyl-guanidin-Hydrochlorid, 21,9 g (0,3 Mol) Methylisothiocyanat und 300 ml Acetonitril wird 2 Stunden bei Rückflußtemperatur gerührt. Nach Abkühlen auf 40°C werden 42 g (0,5 Mol) Natriumhydrogencarbonat dazugegeben und das Gemisch wird weitere 40 Minuten bei Rückflußtemperatur gerührt. Dann wird bei 70°C filtriert und das beim Erkalten des Filtrats und Einengen auf 1/4 des Volumens auskristallisierte Produkt wird durch Absaugen isoliert. Man erhält 33,6 g (78% der Theorie) 4-Methyl-5-methylamino-2,4-dihydro-3H-1,2,4-triazol-3-thion vom Schmelzpunkt 213°C.

Beispiel 3

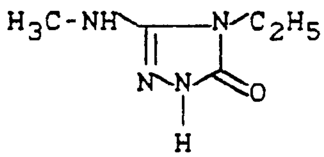

45,75 g (0,3 Mol) 1-Amino-2,2,3-trimethylguanidin-hydrochlorid und 21,3 g (0,3 Mol) Ethylisocyanat werden in 300 ml Acetonitril 2 Stunden bei Rückflußtemperatur gerührt, anschließend auf 40°C abgekühlt, mit 50,4 g (0,6 Mol) Natriumhydrogencarbonat versetzt und weitere 8 Stunden bei Rückflußtemperatur gerührt. Zur Aufarbeitung wird heiß filtriert und das Filtrat abgekühlt. Das ausgefallene Reaktionsprodukt wird abgesaugt, gewaschen und getrocknet.

Man erhält 25 g (59% der Theorie) an 5-Methylamino-4-ethyl-2,4-dihydro-3H-1,2,4-triazol-3-on vom Schmelzpunkt 208-210°C.

Beispiel 4

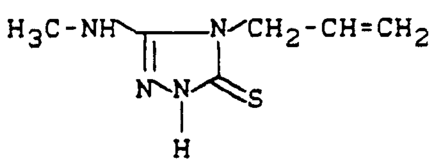

91,5 g (0,6 Mol) 1-Amino-2,2,3-trimethylguanidin-hydrochlorid und 59,4 g (0,6 Mol) Allylisothiocyanat werden in 800 ml Acetonitril 2 Stunden bei Rückflußtemperatur gerührt, anschließend tropfenweise mit 60,6 g (0,6 Mol) Triethylamin versetzt, eine weitere Stunde bei Rückflußtemperatur gerührt, abgekühlt, filtriert, das Filtrat eingeengt und der ölige Rückstand zwischen Dichlormethan und Wasser verteilt. Die vereinigten organischen Phasen werden über Natriumsulfat getrocknet und eingeengt.

Man erhält 68 g (67% der Theorie) an 5-Methylamino-4-allyl-2,4-dihydro-3H-1,2,4-triazol-3-thion vom Schmelzpunkt 129-131°C.

Herstellungsbeispiel für eine herbizidwirksame Verbindung der Formel (V)

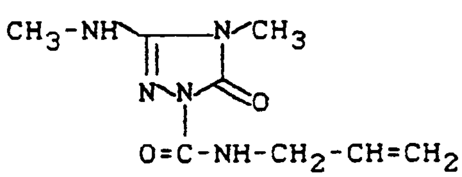

Zu 6,4 g (0,05 Mol) 3-Methylamino-4-methyl-(1H)-1,2,4-triazolin-5-on in 150 ml Acetonitril gibt man 4,2 g (0,05 Mol) Allylisocyanat und 3 Tropfen Diazabicycloundecen (DBU), wobei die Temperatur der Reaktionsmischung auf 30°C ansteigt. Anschließend rührt man 30 Minuten bei Rückflußtemperatur, engt dann im Vakuum ein und kristallisiert aus Essigester um.

Man erhält 7,9 g (75 % der Theorie) an 1-Allylaminocarbonyl-3-methylamino-4-methyl-1,2,4-triazolin-5-on vom Schmelzpunkt 136 - 137°C.

## Patentansprüche

1. Verfahren zur Herstellung von Triazolon-Derivaten der Formel (I)

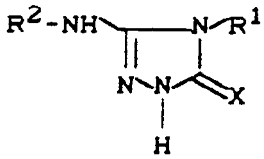

(I)

in welcher

$R^1$ für Alkyl, Alkenyl, Alkinyl, Halogenalkyl, Halogenalkenyl, Halogenalkinyl, Alkoxyalkyl, Cycloalkylalkyl, Cycloalkyl oder für jeweils gegebenenfalls substituiertes Aralkyl oder Aryl steht,

$R^2$ für Alkyl, Alkenyl, Alkinyl, Halogenalkyl, Halogenalkenyl, Halogenalkinyl, Alkoxyalkyl, Cycloalkyl, Cycloalkylalkyl oder für jeweils gegebenenfalls substituiertes Aryl, Aralkyl oder Heteroaryl steht und

X für Sauerstoff oder Schwefel steht,

dadurch gekennzeichnet, daß man Iso(thio)cyanate der allgemeinen Formel (II)

$$X{=}C{=}N{-}R^1 \quad \text{(II)}$$

in welcher

$R^1$ und X die oben angegebenen Bedeutungen haben,

mit Aminoguanidinen der allgemeinen Formel (III)

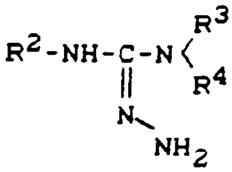

(III)

in welcher

$R^2$ die oben angegebene Bedeutung hat und

$R^3$ und $R^4$ unabhängig voneinander für Alkyl stehen,

oder mit Säureaddukten von Verbindungen der Formel (III)

umsetzt (Stufe 1) und die hierbei gebildeten (Thio)ureidoguanidine der allgemeinen Formel (IV)

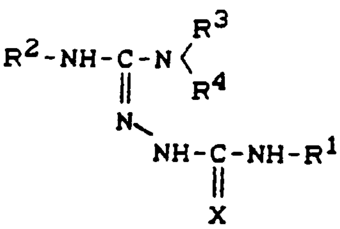

(IV)

in welcher

$R^1$, $R^2$, $R^3$, $R^4$ und X die oben angegebenen Bedeutungen haben,

oder gegebenenfalls Säureaddukte von Verbindungen der Formel (IV)

unter Abspaltung eines Dialkylamins ($HNR^3R^4$) kondensiert.

2. Verfahren gemäß Anspruch 1, dadurch gekennzeichnet, daß man die Umsetzung bei Temperaturen zwischen 0°C und 150°C durchführt.

3. Verfahren gemäß Anspruch 1, dadurch gekennzeichnet, daß man die Umsetzung in Gegenwart eines Lösungsmittels durchführt.

4. Verfahren gemäß Anspruch 1, dadurch gekennzeichnet, daß die Umsetzung in Gegenwart eines Säurebindemittels durchgeführt wird.

5. Verfahren gemäß Anspruch 1, dadurch gekennzeichnet, daß man auf 1 Mol Iso(thio)cyanat der Formel (II) 0,8 bis 1,2 Mol Aminoguanidin der Formel (III) oder ein Säureaddukt hiervon einsetzt.

6. Verfahren gemäß Anspruch 1, dadurch gekennzeichnet, daß man die Reaktion in 2 Stufen durchführt.

7. Verfahren gemäß Anspruch 1, dadurch gekennzeichnet, daß man die Reaktion als Eintopfverfahren durchführt.

8. Verfahren gemäß Anspruch 1, dadurch gekennzeichnet, daß in Formel (I)

$R^1$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten infrage kommen:

Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/oder Schwefel steht, wobei als Substituenten jeweils infrage kommen:

Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, und

X für Sauerstoff oder Schwefel steht.

9. (Thio)ureidoguanidine der Formel (IV)

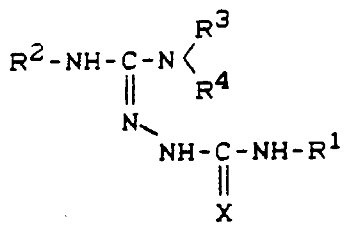

(IV)

in welcher

$R^1$, $R^2$ und X die oben angegebenen Bedeutungen haben und

$R^3$ und $R^4$ unabhängig voneinander für Alkyl stehen.

## Claims

1. Process for the preparation of triazolone derivatives of the formula (I)

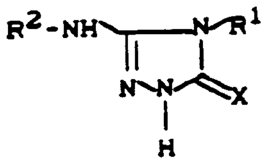

(I)

in which

$R^1$ represents alkyl, alkenyl, alkynyl, halogenoalkyl, halogenoalkenyl, halogenoalkynyl, alkoxyalkyl, cycloalkylalkyl, cycloalkyl or represents aralkyl or aryl, each of which may be substituted if desired,

$R^2$ represents alkyl, alkenyl, alkynyl, halogenoalkyl, halogenoalkenyl, halogenoalkynyl, alkoxyalkyl, cycloalkyl, cycloalkylalkyl or represents aryl, aralkyl or heteroaryl, each of which may be substituted if desired and

X represents oxygen or sulphur,

characterized in that iso(thio)cyanates of the general formula (II)

$$X{=}C{=}N{-}R^1 \quad (II)$$

in which

$R^1$ and X have the meanings given above, are reacted (Step 1) with aminoguanidines of the general formula (III)

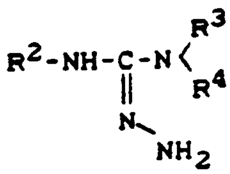

(III)

in which

$R^2$ has the meaning given above and

$R^3$ and $R^4$, independently of one another, represent alkyl, or with acid adducts of compounds of the formula (III) and the (thio)ureidoguanidines formed in this reaction of the general formula (IV)

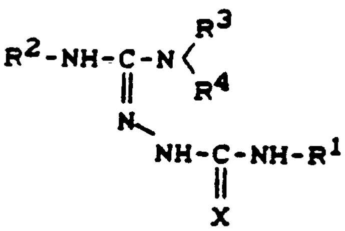

(IV)

in which

$R^1$, $R^2$, $R^3$, $R^4$ and X have the meanings given above, or, if desired, acid adducts of compounds of the formula (IV)

are condensed with elimination of a dialkylamine ($HNR^3R^4$).

2. Process according to Claim 1, characterized in that the reaction is carried out at temperatures between 0°C and 150°C.

3. Process according to Claim 1, characterized in that the reaction is carried out in the presence of a solvent.

4. Process according to Claim 1, characterized in that the reaction is carried out in the presence of an acid-binding agent.

5. Process according to Claim 1, characterized in that 0.8 to 1.2 mol of aminoguanidine of the formula (III) or an acid adduct thereof are used per 1 mol of iso(thio)cyanate of the formula (II).

6. Process according to Claim 1, characterized in that the reaction is carried out in 2 steps.

7. Process according to Claim 1, characterized in that the reaction is carried out as a one-pot process.

8. Process according to Claim 1, characterized in that in the formula (I)

$R^1$ represents alkyl having 1 to 8 carbon atoms, alkenyl having 2 to 8 carbon atoms, alkynyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkynyl having 2 to 8 carbon atoms and 1 to 13 identical or different halogen atoms, and alkoxyalkyl, the individual alkyl components having in each case 1 to 6 carbon atoms and being straight-chain or branched in each case, represents cycloalkylalkyl or cycloalkyl each having 3 to 7 carbon atoms in the cycloalkyl component and if desired 1 to 6 carbon atoms in the straight-chain or branched alkyl component or represents aralkyl or aryl, each of which if desired may be singly or multiply substituted by identical or different substituents, and having in each case 6 to 10 carbon atoms in the aryl component and if desired 1 to 6 carbon atoms in the straight-chain or branched alkyl component, suitable aryl substituents being:

halogen, cyano, nitro, and alkyl, alkoxy, alkylthio, halogenoalkyl, halogenoalkoxy or halogenoalkylthio, each of which may be straight-chain or branched, and having in each case 1 to 6 carbon atoms and if desired 1 to 9 identical or different halogen atoms,

$R^2$ represents alkyl having 1 to 8 carbon atoms, alkenyl having 2 to 8 carbon atoms, alkynyl having 2 to 8 carbon atoms, halogenoalkyl having 1 to 8 carbon atoms and 1 to 17 identical or different halogen atoms, halogenoalkenyl or halogenoalkynyl having in each case 2 to 8 carbon atoms and 1 to 15 or 13 identical or different halogen atoms, and alkoxyalkyl, each having 1 to 6 carbon atoms in the individual alkyl components and being straight-chain or branched in each case, represents cycloalkyl having 3 to 7 carbon atoms, represents cycloalkylalkyl having 3 to 7 carbon atoms in the cycloalkyl component and 1 to 6 carbon atoms in the alkyl component or represents aralkyl having 6 to 10 carbon atoms in the aryl component and 1 to 6 carbon atoms in the alkyl component, aryl having 6 to 10 carbon atoms or heteroaryl having 2 to 9 carbon atoms and 1 to 3 heteroatoms, in particular nitrogen, oxygen and/or sulphur, and each of which may if desired be singly or multiply substituted by identical or different substituents, suitable substituents in each case being: halogen, cyano, nitro and in each case straight-chain or branched alkyl, alkoxy, alkylthio, halogenoalkyl, halogenoalkoxy or halogenoalkylthio, each having 1 to 4 carbon atoms and if desired 1 to 9 identical or different halogen atoms and

X represents oxygen or sulphur.

9. (Thio)ureidoguanidines of the formula (IV)

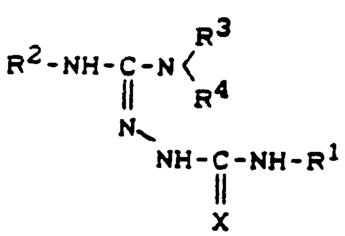

(IV)

in which
$R^1$, $R^2$ and X have the meanings given above and
$R^3$ and $R^4$ independently of each other represent alkyl.

## Revendications

1. Procédé de production de dérivés de triazolones de formule (I)

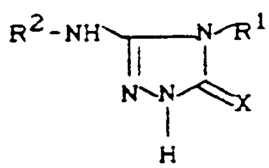

(I)

dans laquelle

$R^1$ est un groupe alkyle, alcényle, alcynyle, halogénalkyle, halogénalcényle, halogénalcynyle, alkoxyalkyle, cycloalkylalkyle, cycloalkyle, ou bien un groupe aralkyle ou aryle chacun éventuellement substitué,

$R^2$ est un groupe alkyle, alcényle, alcynyle, halogénalkyle, halogénalcényle, halogénalcynyle, alkoxyalkyle, cycloalkyle, cycloalkylalkyle ou un groupe aryle, aralkyle ou hétéro-aryle chacun éventuellement substitué et

X représente l'oxygène ou le soufre,

caractérisé en ce qu'on fait réagir (étape 1) des iso(thio)cyanates de formule générale (II)

$$X{=}C{=}N{-}R^1 \qquad \text{(II)}$$

dans laquelle

$R^1$ et X ont les définitions indiquées ci-dessus, avec des aminoguanidines de formule générale (III)

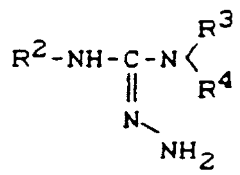

(III)

dans laquelle

$R^2$ a la définition indiquée ci-dessus et

$R^3$ et $R^4$ représentent indépendamment l'un de l'autre un groupe alkyle,

ou avec des produits d'addition d'acides de composés de formule (III) et on condense les (thio)uréidoguanidines ainsi formées, de formule générale (IV)

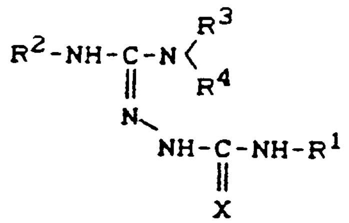

(IV)

dans laquelle

$R^1$ $R^2$, $R^3$, $R^4$ et X ont les définitions indiquées ci-dessus, ou le cas échéant des produits d'addition d'acides de composés de formule (IV)

avec élimination d'une dialkylamine ($HNR^3R^4$).

**2.** Procédé suivant la revendication 1, caractérisé en ce qu'on conduit la réaction à des températures comprises entre 0°C et 150°C.

**3.** Procédé suivant la revendication 1, caractérisé en ce qu'on conduit la réaction en présence d'un solvant.

**4.** Procédé suivant la revendication 1, caractérisé en ce qu'on conduit la réaction en présence d'un accepteur d'acide.

**5.** Procédé suivant la revendication 1, caractérisé en ce qu'on utilise par mole d'iso(thio)cyanate de formule (II) 0,8 à 1,2 mole d'aminoguanidine de formule (III) ou d'un produit d'addition d'acide de ce composé.

**6.** Procédé suivant la revendication 1, caractérisé en ce qu'on conduit la réaction en deux étapes.

**7.** Procédé suivant la revendication 1, caractérisé en ce qu'on conduit la réaction comme un procédé en récipient unique.

**8.** Procédé suivant la revendication 1, caractérisé en ce que dans la formule (I)

$R^1$ est un groupe alkyle ayant 1 à 8 atomes de carbone, un groupe alcényle ayant 2 à 8 atomes de carbone, un groupe alcynyle ayant 2 à 8 atomes de carbone, un groupe halogénalkyle ayant 1 à 8 atomes de carbone et 1 à 17 atomes d'halogènes identiques ou différents, un groupe halogénalcényle ayant 2 à 8 atomes de carbone et 1 à 15 atomes d'halogènes identiques ou différents, un groupe halogénalcynyle ayant 2 à 8 atomes de carbone et 1 à 13 atomes d'halogènes identiques ou différents, un groupe alkoxyalkyle ayant 1 à 6 atomes de carbone dans chacune des parties alkyle individuelles, chacun étant

à chaîne droite ou ramifiée, un groupe cycloalkylalkyle ou cycloalkyle ayant chacun 3 à 7 atomes de carbone dans la partie cycloalkyle et le cas échéant 1 à 6 atomes de carbone dans la partie à chaîne droite ou ramifiée ou un groupe aralkyle ou aryle ayant chacun 6 à 10 atomes de carbone dans la partie aryle et le cas échéant 1 à 6 atomes de carbone dans la partie alkyle à chaîne droite ou ramifiée portant chacun le cas échéant un ou plusieurs substituants identiques ou différents, et on considère alors comme substituants du groupe aryle :

un halogène, un groupe cyano, nitro, ainsi qu'un groupe alkyle, alkoxy, alkylthio, halogénalkyle, halogénalkoxy ou halogénalkylthio, chacun à chaîne droite ou ramifiée, ayant chacun 1 à 4 atomes de carbone et le cas échéant 1 à 9 atomes d'halogènes identiques ou différents,

$R^2$ est un groupe alkyle ayant 1 à 8 atomes de carbone, un groupe alcényle ayant 2 à 8 atomes de carbone, un groupe alcynyle ayant 2 à 8 atomes de carbone, un groupe halogénalkyle ayant 1 à 8 atomes de carbone et 1 à 17 atomes d'halogènes identiques ou différents, un groupe halogénalcényle ou halogénalcynyle ayant chacun 2 à 8 atomes de carbone et 1 à 15 ou respectivement 13 atomes d'halogènes identiques ou différents, un groupe alkoxyalkyle ayant 1 à 6 atomes de carbone dans chaque partie alkyle individuelle, chacun étant à chaîne droite ou ramifiée, un groupe cycloalkyle ayant 3 à 7 atomes de carbone, un groupe cycloalkylalkyle ayant 3 à 7 atomes de carbone dans la partie cycloalkyle et 1 à 6 atomes de carbone dans la partie alkyle, ou un groupe aralkyle ayant 6 à 10 atomes de carbone dans la partie aryle et 1 à 6 atomes de carbone dans la partie alkyle, un groupe aryle ayant 6 à 10 atomes de carbone ou un groupe hétéroaryle ayant 2 à 9 atomes de carbone et 1 à 3 hétéroatomes, notamment d'azote, d'oxygène et/ou de soufre, chacun portant éventuellement un ou plusieurs substituants identiques ou différents, et on considère alors dans chaque cas comme substituants :

un halogène, un groupe cyano, nitro, de même qu'un groupe alkyle, alkoxy, alkylthio, halogénalkyle, halogénalkoxy ou halogénalkylthio, chacun à chaîne droite ou à chaîne ramifiée, ayant chacun 1 à 4 atomes de carbone et le cas échéant 1 à 9 atomes d'halogènes identiques ou différents

et

X est l'oxygène ou le soufre.

9. (Thio)uréidoguanidines de formule (IV)

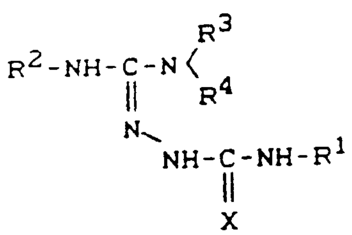

(IV)

dans laquelle

$R^1$, $R^2$ et X ont les définitions indiquées ci-dessus et

$R^3$ et $R^4$ représentent indépendamment l'un de l'autre un groupe alkyle.